# EUROPÄISCHE PATENTANMELDUNG

(11) **EP 3 834 958 A1**
(43) Veröffentlichungstag der Anmeldung: **16.06.2021**
(21) Anmeldenummer: 19215551.3
(22) Anmeldetag: 12.12.2019
(51) Int. Cl.: B21F 15/10, A61B 5/00, A61F 2/95

(54) **BAUGRUPPE AUFWEISEND EINEN METALLDRAHT UND EIN RINGELEMENT**

(71) Anmelder: BIOTRONIK AG, 8180 Bülach (CH)
(72) Erfinder: Fargahi, Amir, 8180 Bülach (CH)
(74) Vertreter: Randoll, Sören

(57) **Zusammenfassung**

Die Erfindung betrifft eine Baugruppe (10), insbesondere für ein medizinisches Gerät, mit: einem Metalldraht (20), und einem Ringelement (30), wobei das Ringelement (30) eine in einer Umfangsrichtung (U) umlaufende Wandung (31) aufweist, die eine umlaufende Stirnseite (32) aufweist, die eine Öffnung (33) des Ringelements (30) begrenzt. Erfindungsgemäß ist vorgesehen, dass der Metalldraht (20) auf der Stirnseite (32) angeordnet und dort festgelegt ist.

## Beschreibung

Die vorliegende Erfindung betrifft eine Baugruppe, insbesondere für ein medizinisches Gerät, umfassend einen Metalldraht und ein Ringelement.

In der Medizintechnologie werden oft Komponenten aus einem Ringelement, das mit einem langen Draht verbunden ist, benötigt.

Als Beispiel kann man das Konzept bei einer Katheeranordnung zur Freigabe von Stents nennen, wobei z.B. ein Stahlring, der auf einem Außenschaft befestigt ist, und ein darauf geschweißtes Zugseil verwendet wird, um den Außenschaft in proximaler Richtung unter Freisetzung eines unter dem Außenschaft befindlichen Stents zu ziehen.

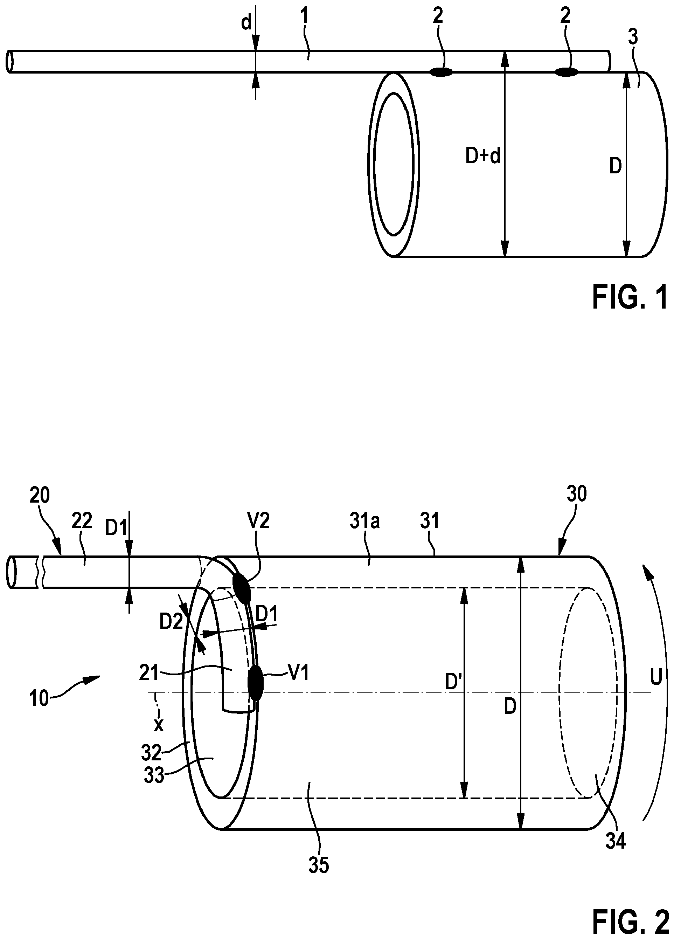

Derartige Verbindungen bzw. Baugruppen haben jedoch regelmäßig eine unerwünschte Vergrößerung des Durchmessers im Bereich der Verbindung zur Folge, wie es in der Figur 1 illustriert ist. Das Festlegen des Metalldrahts 1 auf der Außenseite der Wandung des Ringelements 3 über lokale Verbindungen 2 erhöht den Außendurchmesser D des Ringelements im Bereich der besagten Verbindung auf D+d, wobei d der Durchmesser des Metalldrahts ist.

Hiervon ausgehend liegt der vorliegenden Erfindung daher die Aufgabe zugrunde, eine Baugruppe bereitzustellen, die im Hinblick auf die oben geschilderte Problematik verbessert ist.

Diese Aufgabe wird durch eine Baugruppe mit den Merkmalen des Anspruchs 1 gelöst.

Vorteilhafte Ausgestaltungen der Erfindung sind in den entsprechenden Unteransprüchen angegeben und werden nachfolgend beschrieben.

Gemäß Anspruch 1 wird eine Baugruppe, insbesondere für medizinische Geräte, offenbart, mit:
- einem Metalldraht, und
- einem Ringelement, das eine in einer Umfangsrichtung des Ringelements umlaufende Wandung aufweist, die eine entsprechend umlaufende Stirnseite aufweist, die eine Öffnung des Ringelements begrenzt.
Erfindungsgemäß ist dabei vorgesehen, dass der Metalldraht auf der Stirnseite angeordnet und dort festgelegt ist.

Der Metalldraht kann eines der folgenden Metalle oder Metalllegierungen aufweisen oder kann aus einem der folgenden Metalle oder Metalllegierungen gebildet sein: Eisen, Kupfer, Messing, Aluminium, Silber, Gold, Chrom, Kobalt, Platin, Nickel, Nitinol, Tantal, Wolfram, Magnesium, rostfreier Stahl und sowie unterschiedlichste Kupferlegierungen. Ferner können auch Komposite verwendet werden, die zum Beispiel als DFT-Draht (Handelsname, drawn filled tubing) bekannt sind.

Weiterhin kann das Ringelement aus jedem geeigneten Material hergestellt sein, insbesondere aus einem Metall oder einer Metalllegierung. Die Ausgestaltung als Metall oder Metalllegierung hat den Vorteil, dass die Verbindung zwischen Ring und Draht durch eine Verlötung oder Verschweißung vorgenommen werden kann. Ferner kann es von Vorteil sein, wenn das Ringelement aus einem röntgensichtbaren Material hergestellt ist. Beispiele für röntgensichtbare Metalle sind Gold, Platin, Iridium, Tantal, Wolfram, Rhenium, Osmium, Blei und Legierungen daraus.

Des Weiteren sei angemerkt, dass das Ringelement auch eine andere geometrische Figur im Querschnitt bilden kann als einen Kreis. Es ist auch denkbar, dass das Ringelement einen ovalen, ellipsoiden, dreieckigen, viereckigen oder quadratischen Querschnitt aufweisen kann. Das Ringelement wurde als "Ring" bezeichnet, da eine kreisrunde Ausführungsform die wahrscheinlichste ist. Dies soll aber die hierin vorgestellte Baugruppe nicht auf eine bestimmte Querschnittsform einschränken.

Gemäß einer Ausführungsform der Erfindung ist vorgesehen, dass der Metalldraht einen Endabschnitt aufweist.

Weiterhin ist gemäß einer Ausführungsform der Erfindung vorgesehen, dass der Endabschnitt des Metalldrahts auf der Stirnseite angeordnet ist und der Metalldraht über den Endabschnitt an der Stirnseite festgelegt ist.

Weiterhin ist gemäß einer Ausführungsform der Erfindung vorgesehen, dass der Endabschnitt einer Krümmung der Stirnseite folgt. Gemäß einer Ausführungsform ist der Endabschnitt vorzugsweise kreisbogenförmig ausgebildet.

Bei einem kreisbogenförmig ausgestalteten bzw. gekrümmte Endabschnitt ist gemäß einer Ausfiihrungsform vorzugsweise vorgesehen, dass ein entsprechender Kreisbogenwinkel vorzugsweise zumindest 45° beträgt, vorzugsweise zumindest 90°, d.h., dass sich der Endabschnitt des Metalldrahts zumindest über ein Achtel der Stirnseite in Umfangsrichtung der Wandung erstreckt, vorzugsweise zumindest über ein Viertel der Stirnseite in Umfangsrichtung der Wandung.

Gemäß einer bevorzugten Ausführungsform ist weiterhin vorgesehen, dass der Endabschnitt des Metalldrahts in Richtung einer Normalen zur Außenseite der Wandung nicht über die Außenseite der Wandung hinausragt und/oder dass der Endabschnitt des Metalldrahts in Richtung einer Normalen zu einer Innenseite der Wandung nicht über die Innenseite der Wandung hinausragt. Die jeweilige Normale steht dabei senkrecht auf der Außen- bzw. Innenseite der Wandung und verläuft jeweils senkrecht zu einer axialen Richtung des Ringelementes, die insbesondere mit einer Zylinderachse des Ringelements zusammenfällt.

Weiterhin ist gemäß einer Ausführungsform der Erfindung vorgesehen, dass der Endabschnitt des Metalldrahts einen Durchmesser aufweist, der kleiner oder gleich einer Wanddicke der Wandung des Ringelements ist.

Gemäß einer bevorzugten Ausführungsform der Erfindung ist weiterhin vorgesehen, dass das Ringelement ein Hohlzylinder ist.

Der Querschnitt des Metalldrahts, insbesondere des Endabschnitts, ist gemäß einer Ausfiihrungsform vorzugsweise kreisförmig.

Gemäß einer bevorzugten Ausführungsform der Erfindung ist weiterhin vorgesehen, dass die Stirnseite einen Kreisring bildet, wobei insbesondere der Durchmesser des ersten Endabschnitts des Metalldrahts kleiner oder gleich der Ringbreite des Kreisrings ist, wobei die Ringbreite hier insbesondere der Wanddicke der Wandung des Ringelements entspricht.

Weiterhin ist gemäß einer Ausführungsform der Erfindung vorgesehen, dass der Metalldraht einen mit dem Endabschnitt verbundenen Abschnitt aufweist, der winklig zu dem Endabschnitt verläuft. Dieser Abschnitt verläuft gemäß einer Ausführungsform der Erfindung bevorzugt in einer axialen Richtung des Ringelements, wobei die axiale Richtung senkrecht zur Ebene verläuft, in der die Wandung des Ringelements in der Umfangsrichtung umläuft. Der sich an den Endabschnitt anschließende Abschnitt des Metalldrahts kann zumindest abschnittsweise von einem elektrisch isolierenden Material umgeben sein, z.B. Polyurethan.

Weiterhin ist gemäß einer Ausführungsform der Erfindung vorgesehen, dass der Endabschnitt des Metalldrahts über zumindest eine Verbindung mit der Stirnseite verbunden ist. Die mindestens eine Verbindung kann sich dabei über eine gesamte Länge oder einen Großteil der Länge des Endabschnitts des Metalldrahts erstrecken und kann z.B. als eine entsprechend längserstreckte geschweißte Verbindung bzw. Schweißnaht oder eine längserstreckte gelötete Verbindung ausgebildet sein.

Gemäß einer bevorzugten Ausführungsform ist vorgesehen, dass der Endabschnitt des Metalldrahts einen ersten Bereich aufweist, der ein Ende des Metalldrahtes aufweist sowie einen zweiten Bereich, an den sich der besagte Abschnitt des Metalldrahtes anschließt, der winklig zu dem Endabschnitt des Metalldrahtes verläuft bzw. sich entlang der axialen Richtung erstreckt (siehe oben).

Gemäß einer Ausführungsform der Erfindung ist vorgesehen, dass der Endabschnitt des Metalldrahts über eine erste Verbindung und eine separate zweite Verbindung mit der Stirnseite der Wandung des Ringelements verbunden ist, wobei insbesondere die jeweilige Verbindung zumindest abschnittsweise zwischen dem Endabschnitt des Metalldrahts und der Stirnseite des Ringelements angeordnet ist. Bevorzugt verbindet die erste Verbindung den ersten Bereich des Endabschnitts des Metalldrahts mit der Stirnseite der Wandung des Ringelements, wohingegen die zweite Verbindung den zweiten Bereich des Endabschnitts des Metalldrahts mit der Stirnseite der Wandung des Ringelements verbindet.

Die mindestens eine Verbindung bzw. die jeweilige oben beschriebene Verbindung ist vorzugsweise eine geschweißte Verbindung, z.B. in Form einer Schweißnaht, oder eine gelötete Verbindung. Die jeweilige geschweißte Verbindung kann eine Laserschweißverbindung sein, d.h., eine durch Laserschweißen hergestellte geschweißte Verbindung. Die jeweilige gelötete Verbindung besteht vorzugsweise aus einem geeigneten Lotmaterial.

Die heirin vorgeschlagene Baugruppe kann grundsätzlich überall zum Einsatz kommen, wo eine Platzersparnis von Nutzen ist. Dies kann in Werkzeugen, Fahrzeugen, Flugzeugen Bergwerken, Uhren, Robotern oder auch Bauwerken, um einige zu nennen der Fall sein.

Ein weiterer Aspekt der vorliegenden Erfindung betrifft ein medizinisches Gerät, aufweisend eine erfindungsgemäße Baugruppe. Als medizinisches Gerät kommen insbesondere Implantate oder medizinische Geräte, die in den Körper eines Patienten eingeführt werden müssen, in Frage. Hier ist eine Platzersparnis von besonderem Vorteil. Beispiele für medizinische Geräte sind Katheteranordnungen, Herzschrittmacher, sowie Elektroden dafür, Defibrillatoren und Stents, insbesondere mit Sensoren.

Bei Katheeranordnungen, die für einen koronaren Einsatz vorgesehen sind kann eine Durchmesserersparnis insbesondere von Vorteil sein, da hier selbst Bruchteil von Millimetern ein Vorteil bei der Einführung in das Gefässsystem bringen. Die hierin vorgeschlagene Baugruppe kann dabei zum Beispiel bei der Verbindung von einem Zugdraht mit einer Hülse zum Einsatz kommen. Die Hülse kann dabei mit einem Außenschaft verbunden sein, der durch den Zugdraht in proximale Richtung gezogen wird.

Ein weiteres Einsatzgebiet kann innerhalb einer Katheteranordnung gegeben sein, wenn röntgensichtbarer Marker in einem Ballonkatheter mit einem Leiter verbunden werden soll. Ein solcher Marker kann ferner als Sensor verwendet werden, um eine Signal zu generieren, anhand dessen der Grad der Inflation des Katheterballons gemessen werden kann. Für einen solchen Einsatz ist die Ersparnis von Bruchteilen eines Millimeters von Vorteil.

Im Folgenden sollen Ausführungsformen sowie weitere Merkmale und Vorteile der Erfindung anhand der Figuren erläutert werden. Es zeigen:
- Fig. 1: eine aus dem Stand der Technik bekannte Baugruppe, bei der ein Endabschnitt eines Metalldrahts auf einer Außenseite einer Wandung eines Ringelements festgelegt ist, was zu einer unerwünschten Durchmesservergrößerung im Bereich des Endabschnitts des Metalldrahts führt; und
- Fig. 2: eine Ausführungsform einer erfindungsgemäßen Baugruppe.

Figur 2 zeigt eine Ausführungsform einer erfindungsgemäßen Baugruppe 10. Die Baugruppe 10 weist einen Metalldraht 20 auf, der abschnittsweise isoliert sein kann (z.B. mittels einer Ummantelung aus einem elektrisch isolierenden Material, wie z. B. Polyurethan), und ein Ringelement 30, das z. B. hohlzylindrisch ausgebildet sein kann und dabei einen Außendurchmesser D aufweist. Das Ringelement 30 weist eine in einer Umfangsrichtung U umlaufende Wandung 31 auf, die eine in der Umfangsrichtung U umlaufende Stirnseite 32 aufweist, wobei die Stirnseite 32 eine Öffnung 33 des Ringelements 30 begrenzt, und vorzugsweise als Kreisring ausgestaltet ist. Der Öffnung 33 liegt in der axialen Richtung x eine weitere Öffnung 34 gegenüber, so dass sich zwischen diesen beiden Öffnungen 33, 34 ein durchgängiger Innenraum 35 des Ringelements 30 erstreckt, wobei eine Innenseite 31b der Wandung 30 diesem Innenraum 35 zugewandt ist. Die Wandung 30 weist des Weiteren eine der Innenseite 31b abgewandte Außenseite 31a auf, wobei Innen- und Außenseite 31b, 31a über die besagte Stirnseite 32, die parallel zur Öffnung 33 verläuft, miteinander verbunden sind.

Erfindungsgemäß ist nun vorgesehen, dass der Metalldraht 20 auf der Stirnseite 32 angeordnet und dort festgelegt ist. Gegenüber der in der Figur 1 gezeigten bekannten Anordnung hat dies den Vorteil, dass der Metalldraht 20 gemäß Figur 2 nicht den Durchmesser D der gesamten Baugruppe 10 vergrößert, wie es gemäß Figur 1 der Fall ist, wobei aufgrund der Anordnung des Metalldrahts 20 an der Stirnseite 32 dennoch eine stabile Verbindung zwischen Metalldraht 20 und Ringelement 30 möglich ist.

Bei dem Metalldraht 20 kann es sich um einen Kupfer-Draht handeln, der abschnittsweise mit Polyurethan isoliert sein kann, wobei der Durchmesser d (z.B. im Bereich des Abschnitts 22) z. B. 0,05 mm betragen kann. Bei dem Ringelement 30 kann es sich um einen Röntgenmarker-Ring aus einer Pt-Ir-Legierung handeln, der eine Wanddicke D2 von z.B. 0,05 mm, einen Innendurchmesser von D'=0,45 mm und einen Außendurchmesser von D=0,55 mm aufweisen kann.

Der Metalldraht 20 ist vorzugsweise über einen Endabschnitt 21 an der Stirnseite 32 festgelegt, wobei der Endabschnitt 21 so gekrümmt ist (hier kreisbogenförmig), dass der Endabschnitt 21 einer Krümmung der Stirnseite 32 folgt gemäß Figur 2 folgt. Hierbei erstreckt sich der Endabschnitt 21 in Umfangsrichtung U etwa entlang eines Viertels der Stirnseite 32. Dabei ragt der Endabschnitt 21 nach innen und nach außen nicht über die Innenseite 31b und die Außenseite 31a hinaus. Vorzugsweise entspricht ein Durchmesser D1 des Endabschnitts daher der Wanddicke D2 oder ist geringer als diese ausgebildet.

Der Metalldraht 20 trägt somit im Bereich des Ringelements 30 nicht zum Gesamtaußendurchmesser D des Ringelements 30 bzw. der Baugruppe 10 bei, so dass die Baugruppe 10 in radialer Richtung des Ringelements 30 kleinbauend ist und des Weiteren die volle Öffnungsfläche 33 zugänglich ist. Der Endabschnitt 21 des Metalldrahts 20 ist mit einem weiteren Abschnitt 22 des Metalldrahts 20 verbunden, der vorzugsweise entlang der axialen Richtung x verläuft und entsprechend winklig zum Endabschnitt 21 angeordnet ist. Dieser Abschnitt 22 kann z.B. mit der elektrisch isolierenden Ummantelung umgeben sein (z.B. PUR).

Gemäß der in der Figur 2 gezeigten Ausführungsform kann der Endabschnitt 21 des Metalldrahts 20 über zwei separate punktuelle Verbindungen V1, V2, die hier als Laserschweißverbindungen ausgestaltet sind, mit der Stirnseite 32 der Wandung 30 des Ringelements 31 verbunden sein. Die entsprechenden Schweißnähte V1, V2 sind dabei zumindest abschnittsweise (bezogen auf die axiale Richtung x) zwischen dem Endabschnitt 21 des Metalldrahts 20 und der Stirnseite 32 des Ringelements 30 angeordnet.

Die vorliegende Erfindung ermöglicht eine sichere Verbindung zwischen einem Ringelement 30 und einem Draht 20 ohne Durchmesservergrößerung, wobei gleichzeitig eine optimale mechanische Festigkeit der Verbindung erzielbar ist, sowie ein optimaler elektrischer und mechanischer Kontakt zwischen dem Ringelement 30 und dem Draht 20. Da der Metalldraht 20 nicht auf der Außenseite 31a des Ringelements 30 verläuft, kann das Ringelement 30 ferner auf einfache Weise durch mechanisches Krimpen (Swaging) weiterverarbeitet werden.

## Patentansprüche

1. Baugruppe (10), mit:
- einem Metalldraht (20), und
- einem Ringelement (30), wobei das Ringelement (30) eine in einer Umfangsrichtung (U) des Ringelements (30) umlaufende Wandung (31) aufweist, die eine in der Umfangsrichtung (U) umlaufende Stirnseite (32) aufweist, wobei die Stirnseite (32) eine Öffnung (33) des Ringelements (30) begrenzt,
**dadurch gekennzeichnet,**
**dass** der Metalldraht (20) auf der Stirnseite (32) angeordnet und dort festgelegt ist.

2. Baugruppe nach Anspruch 1, **dadurch gekennzeichnet, dass** der Metalldraht (20) einen Endabschnitt (21) aufweist.

3. Baugruppe nach Anspruch 1 oder 2, **dadurch gekennzeichnet, dass** der Endabschnitt (21) auf der Stirnseite (32) angeordnet ist und der Metalldraht (20) über den Endabschnitt (21) an der Stirnseite (32) festgelegt ist.

4. Baugruppe nach Anspruch 2 oder 3, **dadurch gekennzeichnet, dass** der Endabschnitt (21) des Metalldrahts (20) einer Krümmung der Stirnseite (32) folgt.

5. Baugruppe nach einem der Ansprüche 2 bis 4, **dadurch gekennzeichnet, dass** der Endabschnitt (21) des Metalldrahts (20) senkrecht zu einer Außenseite (31a) der Wandung (30) nicht über die Außenseite (31a) hinausragt.

6. Baugruppe nach einem der Ansprüche 2 bis 5, **dadurch gekennzeichnet, dass** der Endabschnitt (21) des Metalldrahts (20) senkrecht zu einer Innenseite (31b) der Wandung (30) nicht über die Innenseite (31b) hinausragt.

7. Baugruppe nach einem der Ansprüche 2 bis 6, **dadurch gekennzeichnet, dass** der Endabschnitt (21) des Metalldrahts (20) einen Durchmesser (D1) aufweist, der kleiner oder gleich einer Wanddicke (D2) der Wandung (31) ist.

8. Baugruppe nach einem der vorhergehenden Ansprüche, **dadurch gekennzeichnet, dass** das Ringelement (30) als Hohlzylinder ausgestaltet ist.

9. Baugruppe nach einem der vorhergehenden Ansprüche, **dadurch gekennzeichnet, dass** die Stirnseite (32) kreisringförmig ausgebildet ist.

10. Baugruppe nach einem der Ansprüche 2 bis 9, **dadurch gekennzeichnet, dass** der Metalldraht (20) einen mit dem Endabschnitt (21) verbundenen Abschnitt (22) aufweist, der winklig zu dem Endabschnitt (21) verläuft, wobei der Abschnitt (22) insbesondere von einem elektrisch isolierenden Material umgeben ist.

11. Baugruppe nach einem der Ansprüche 2 bis 10, **dadurch gekennzeichnet, dass** der Endabschnitt (21) des Metalldrahts (20) über zumindest eine Verbindung (V1; V2) mit der Stirnseite (32) der Wandung (31) des Ringelements (30) verbunden ist.

12. Baugruppe nach einem der Ansprüche 2 bis 11, **dadurch gekennzeichnet, dass** der Endabschnitt (21) des Metalldrahts (20) über eine erste Verbindung (V1) und über eine separate zweite Verbindung (V2) mit der Stirnseite (32) verbunden ist.

13. Baugruppe nach Anspruch 11 oder 12, **dadurch gekennzeichnet, dass** die jeweilige Verbindung (V1, V2) eine geschweißte Verbindung oder eine gelötete Verbindung ist.

14. Medizinisches Gerät, aufweisend eine Baugruppe (10) nach einem der vorhergehenden Ansprüche.
